# EUROPÄISCHE PATENTSCHRIFT

(11) **EP 3 500 768 B1**
(45) Veröffentlichungstag und Bekanntmachung des Hinweises auf die Patenterteilung: **23.09.2020**
(21) Anmeldenummer: 17757678.2
(22) Anmeldetag: 08.08.2017
(51) Int. Cl.: F16D 7/10, F16D 43/208, F16C 17/04

(54) **DREHMOMENTBEGRENZER**
TORQUE LIMITER
LIMITEUR DE COUPLE

(30) Priorität: 19.08.2016 DE 202016104553 U
(43) Veröffentlichungstag der Anmeldung: 26.06.2019
(73) Patentinhaber: Witte, Peter, 24111 Kiel (DE)
(72) Erfinder: Witte, Peter, 24111 Kiel (DE)
(74) Vertreter: Seemann & Partner Patentanwälte mbB
(86) Internationale Anmeldenummer: PCT/DE2017/100669
(87) Internationale Veröffentlichungsnummer: WO 2018/033179

(56) Entgegenhaltungen:
- DE-U1-202006 004 027
- FR-A- 1 220 285
- JP-A- H0 735 170
- JP-A- H06 341 454

## Beschreibung

Die Erfindung betrifft einen Drehmomentbegrenzer, insbesondere einen Drehmomentbegrenzer zum Begrenzen des Drehmoments eines chirurgischen Schraubendrehers mit den Merkmalen des Oberbegriffs von Anspruch 1.

Ein derartiger Drehmomentbegrenzer für einen chirurgischen Schraubendreher ist aus der DE 20 2006 004 027 bekannt. Bei diesem Drehmomentbegrenzer stellt sich das Problem, dass die Elastizität des als elastomerer O-Ring ausgebildeten elastischen Elements erheblich streut und sich über die Zeit, insbesondere nach thermischer Belastung, ändert.

Der Erfindung liegt die Aufgabe zu Grunde einen Drehmomentbegrenzer zu schaffen, bei dem das maximal übertragbare Drehmoment mit größerer Genauigkeit bestimmt werden kann und sich auch nach thermischer Belastung des Drehmomentbegrenzers - etwa bei dessen Sterilisation - nicht ändert.

Erfindungsgemäß wird diese Aufgabe gelöst durch die Merkmale des kennzeichnenden Teils des Anspruchs 1. Die Unteransprüche geben bevorzugte Ausführungsformen an.

Erfindungsgemäß ist ein Drehmomentbegrenzer vorgesehen, insbesondere für einen chirurgischen Schraubendreher, mit einer Außenhülse, einer in dieser drehgesichert angeordneten Sprenghülse; einem in dieser angeordneten Wälzkörperkäfig, einer in diesem angeordneten Innenhülse und einer von dieser drehgesichert aufgenommenen kraftübertragenden Welle, wobei die Außenhülse, deren Innenwandung mit sich achsparallel erstreckenden Ausnehmungen versehen ist, der Wälzkörperkäfig mit einer Mehrzahl von jeweils einen Wälzkörper haltenden Wälzkörperaufnahmen und einer der Anzahl von Ausnehmungen entsprechenden Anzahl von in die Ausnehmungen eingreifenden Nasen, deren Breite geringer als die lichte Weite der Ausnehmungen ist, versehen ist, und die Innenhülse mit einer Mehrzahl von sich achsparallel erstreckenden V-förmigen, zur Aufnahme der Wälzkörper dienenden Kerben versehen ist, so dass ein auf die Außenhülse in Andrehrichtung aufgebrachtes Drehmoment die einen Seiten der Nasen gegen die eine der Wandungen der Ausnehmungen in eine Position drückt, in der die gegenüber den Nasen leicht versetzt angeordneten Wälzkörper mit den Ausnehmungen fluchten und so bei Erreichen eines maximal zulässigen Drehmoments ein Austreten der Wälzkörper aus den V-förmigen Kerben der Innenhülse in die Ausnehmungen der Außenhülse unter Aufspreizung der Sprenghülse erlaubt und damit die Innenhülse entkoppelt wird, und ein auf die Außenhülse in Ausdrehrichtung aufgebrachtes Drehmoment die anderen Seiten der Nasen gegen die andere der Wandungen der Ausnehmungen in eine Position drückt, in der die Wälzkörper mit den zwischen den Ausnehmungen verbliebenen Stegen fluchten und so ein Austreten der Wälzkörper aus den V-förmigen Kerben der Innenhülse verhindert wird und damit die Innenhülse koppelt.

Durch diese Kopplung erfolgt ein Kraftschluss zwischen der Außenhülse und der Innenhülse. Durch diesen Mechanismus ist eine unbegrenzte Kraftübertragung in Ausdrehrichtung gewährleistet. In dieser Position ist die Sprenghülse von jeglichen Krafteinwirkungen entkoppelt. Dadurch sind eine hohe Funktionsdauer des Drehmomentbegrenzers insgesamt, eine gleichbleibende Genauigkeit und somit die Reproduzierbarkeit der Auslösemomente gewährleistet.

Bevorzugt wird die Drehsicherung zwischen Außenhülse und Sprenghülse durch die Vorsehung eines radial nach innen ragenden, in den Schlitz in der Sprenghülse eingreifenden Mitnehmers an der Innenwandung der Außenhülse bewirkt.

Weiter bevorzugt sind die Wälzkörper als Nadeln und die Wälzkörperaufnahmen als Schlitze ausgebildet.

Speziell ist die Welle nach einer noch weiteren bevorzugten Ausgestaltung an ihrer Spitze mit einem in einer Lagerkappe gelagerten Kegel versehen.

Schließlich ist es vorteilhaft, wenn die Sprenghülse aus einem Polyetheretherketon (PEEK) gefertigt ist.

Die Erfindung wird im Folgenden anhand einer Zeichnung erläutert. Dabei zeigt:

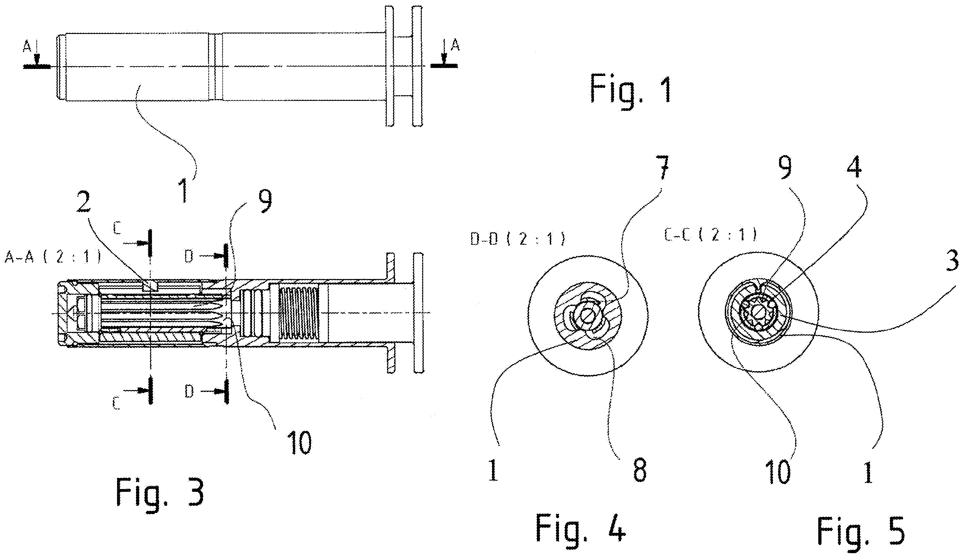

- Fig. 1: eine Außenansicht des Drehmomentbegrenzers,

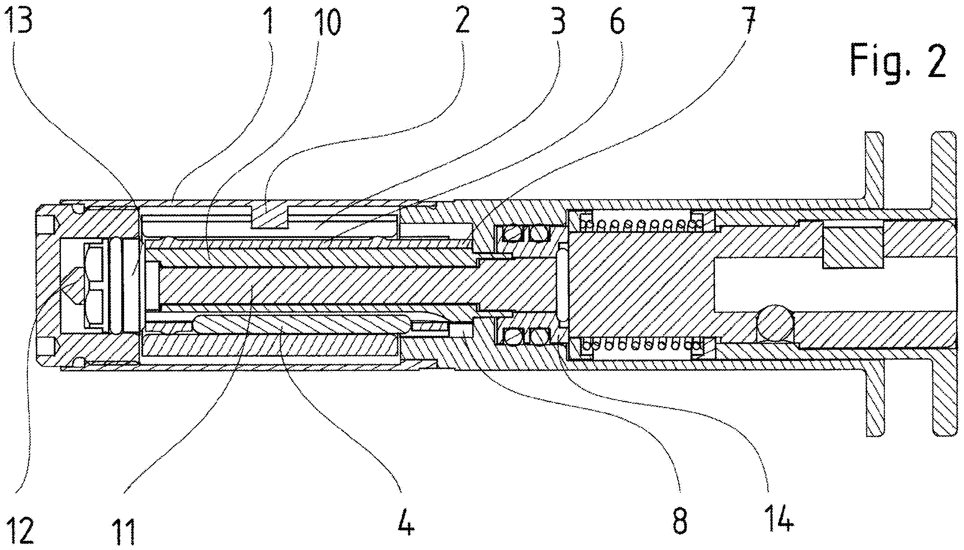

- Fig. 2: eine Längsschnittansicht des Drehmomentbegrenzers entlang der Linie A - A von Fig. 1,
- Fig. 3: eine Fig. 2 entsprechende Längsschnittansicht des Drehmomentbegrenzers unter Darstellung der Innenhülse,
- Fig. 4: eine Schnittansicht entlang der Linie D-D von Fig. 3,
- Fig. 5: eine Schnittansicht entlang der Linie C-C von Fig. 3 und

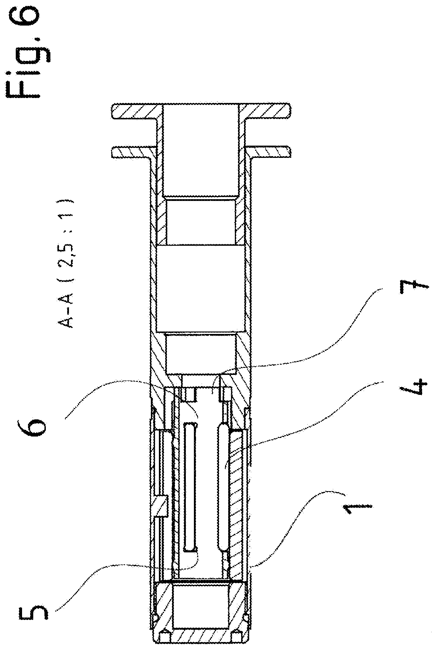

- Fig. 6: eine Fig. 3 entsprechende Längsschnittansicht unter Darstellung des Wälzkörperkäfigs.

Die Figuren zeigen Drehmomentbegrenzer, insbesondere für einen chirurgischen Schraubendreher, mit einer Außenhülse 1, einer in dieser drehgesichert angeordneten Sprenghülse 3; einem in dieser angeordneten Wälzkörperkäfig 6, einer in diesem angeordneten Innenhülse 10 und einer von dieser drehgesichert aufgenommenen kraftübertragenden Welle 11.

Die Innenwandung der Außenhülse 1 ist mit sich achsparallel erstreckenden Ausnehmungen 8 versehen. Der Wälzkörperkäfig 6 ist mit einer Mehrzahl von jeweils einen Wälzkörper 4 haltenden Wälzkörperaufnahmen 5 und einer der Anzahl von Ausnehmungen 8 entsprechenden Anzahl von in die Ausnehmungen 8 eingreifenden Nasen 7, deren Breite geringer als die lichte Weite der Ausnehmungen 8 ist und die gegenüber den Wälzkörperaufnahmen 5 leicht versetzt angeordnet sind, versehen. Die Innenhülse 10 weist eine Mehrzahl von sich achsparallel erstreckenden V-förmigen Kerben 9 auf, die zur Aufnahme der Wälzkörper 4 dienen.

Ein auf die Außenhülse 1 in Andrehrichtung aufgebrachtes Drehmoment drückt die einen Seiten der Nasen 7 gegen die eine der Wandungen der Ausnehmungen 8 in eine Position, in der die Wälzkörper 4 mit den Ausnehmungen 8 fluchten und so bei Erreichen eines maximal zulässigen Drehmoments ein Austreten der Wälzkörper 4 aus den V-förmigen Kerben 9 der Innenhülse 10 in die Ausnehmungen 8 der Außenhülse 1 unter Aufspreizung der Sprenghülse 3 erlaubt und damit die Innenhülse 10 entkoppelt wird.

Ein auf die Außenhülse 1 in Ausdrehrichtung aufgebrachtes Drehmoment drückt die anderen Seiten der Nasen 7 gegen die andere der Wandungen der Ausnehmungen 8 in eine Position, in der die Wälzkörper 4 mit den zwischen den Ausnehmungen 8 verbliebenen Stegen fluchten und so ein Austreten der Wälzkörper 4 aus den V-förmigen Kerben 9 der Innenhülse 10 verhindert und damit die Innenhülse 10 gekopppelt wird.

Die Drehsicherung zwischen Außenhülse 1 und Sprenghülse 3 wird bei dem dargestellten Ausführungsbeispiel durch die Vorsehung eines radial nach innen ragenden, in den Schlitz in der Sprenghülse 3 eingreifenden Mitnehmer 2 an der Innenwandung der Außenhülse 1 bewirkt.

Die Wälzkörper 4 sind hier als Nadeln und die Wälzkörperaufnahmen 5 als Schlitze ausgebildet.

Die Welle 11, die bei Verwendung für ein chirurgisches Instrument an ihrem aus der Aufnahmehülse austretenden Ende das Instrument aufnimmt, ist an ihrem anderen Ende mit einem in einer Lagerkappe gelagerten Kegel 12 versehen. Die Spitze des Kegels 12 ist dabei gegen die Lagerkappe gerichtet und berührt diese bei der praktischen Anwendung.

Durch den in der Lagerkappe bei Verwendung des Drehmomentbegrenzers gelagerten Kegel 12 wird ein nahezu reibungsfreies Lagern zwischen der Außenhülse 1 und der Innenhülse 10 erreicht. Durch dieses konstruktive Merkmal werden die Reibungsmomente minimiert und eine größere Genauigkeit und somit eine geringere Streuung des eingestellten Drehmoments ermöglicht.

Als Material für die Sprenghülse 3 bietet sich Polyetheretherketon PEEK an, dieser Kunststoff ist unter Beibehaltung seiner Elastizität gut sterilisierbar.

## Patentansprüche

1. Drehmomentbegrenzer, insbesondere für einen chirurgischen Schraubendreher, mit
- einer Außenhülse (1), einer in dieser drehgesichert angeordneten Sprenghülse (3); einem in dieser angeordneten Wälzkörperkäfig (6), einer in diesem angeordneten Innenhülse (10) und einer von dieser drehgesichert aufgenommenen kraftübertragenden Welle (11),
wobei
- die Außenhülse (1), deren Innenwandung mit sich achsparallel erstreckenden Ausnehmungen (8) versehen ist,
- der Wälzkörperkäfig (6) mit einer Mehrzahl von jeweils einen Wälzkörper (4) haltenden Wälzkörperaufnahmen (5) und einer der Anzahl von Ausnehmungen (8) entsprechenden Anzahl von in die Ausnehmungen (8) eingreifenden Nasen (7), deren Breite geringer als die lichte Weite der Ausnehmungen (8) ist, versehen ist, und
- die Innenhülse (10) mit einer Mehrzahl von sich achsparallel erstreckenden V-förmigen, zur Aufnahme der Wälzkörper (4) dienenden Kerben (9) versehen ist,
so dass
- ein auf die Außenhülse (1) in Andrehrichtung aufgebrachtes Drehmoment die einen Seiten der Nasen (7) gegen die eine der Wandungen der Ausnehmungen (8) in eine Position drückt, in der die gegenüber den Nasen (7) leicht versetzt angeordneten Wälzkörper (4) mit den Ausnehmungen (8) fluchten und so bei Erreichen eines maximal zulässigen Drehmoments ein Austreten der Wälzkörper (4) aus den V-förmigen Kerben (9) der Innenhülse (10) in die Ausnehmungen (8) der Außenhülse (1) unter Aufspreizung der Sprenghülse (3) erlaubt und damit die Innenhülse (10) entkoppelt wird, und
- ein auf die Außenhülse (1) in Ausdrehrichtung aufgebrachtes Drehmoment die anderen Seiten der Nasen (7) gegen die andere der Wandungen der Ausnehmungen (8) in eine Position drückt, in der die Wälzkörper (4) mit den zwischen den Ausnehmungen (8) verbliebenen Stegen fluchten und so ein Austreten der Wälzkörper (4) aus den V-förmigen Kerben (9) der Innenhülse (10) verhindert wird und damit die Innenhülse (10) koppelt, wobei
- die Welle (11) an ihrer Spitze mit einem in einer Lagerkappe gelagerten Kegel (12) versehen ist.

2. Drehmomentbegrenzer nach Anspruch 1, **dadurch gekennzeichnet, dass** die Drehsicherung zwischen Außenhülse (1) und Sprenghülse (3) durch die Vorsehung eines radial nach innen ragenden, in den Schlitz in der Sprenghülse (3) eingreifenden Mitnehmer (2) an der Innenwandung der Außenhülse (1) bewirkt wird.

3. Drehmomentbegrenzer nach Anspruch 1 oder 2, **dadurch gekennzeichnet, dass** die Wälzkörper (4) Nadeln und die Wälzkörperaufnahmen (5) Schlitze sind.

4. Drehmomentbegrenzer nach einem der vorangehenden Ansprüche, **dadurch gekennzeichnet, dass** die Sprenghülse (3) aus einem Polyetheretherketon (PEEK) gefertigt ist.

## Claims

1. A torque limiter, in particular for a surgical screwdriver, comprising
- an outer sleeve (1), a snap sleeve (3) which is rotationally fixed in the outer sleeve (1); a rolling element cage (6) which is arranged in the snap sleeve (3), an inner sleeve (10) which is arranged in the rolling element cage (6) and a force-transmitting shaft (11) which is received by the inner sleeve (10) in a rotationally fixed manner,
wherein
- the inner wall of the outer sleeve (1) is provided with recesses (8) extending parallel to the axis,
- the rolling element cage (6) is provided with a plurality of rolling element receiving areas (5), each of which holds a rolling element (4), and with a number of noses (7) which engage into the recesses (8) and the width of which is less than the open width of the recesses (8), said number corresponding to the number of recesses (8), and
- the inner sleeve (10) is provided with a plurality of V-shaped notches (9) which extend parallel to the axis and which are used to receive the rolling elements (4)
such that
- a torque applied to the outer sleeve (1) in the screwing direction presses one side of the noses (7) against one of the walls of the recesses (8) into a position in which the rolling elements (4) that are slightly offset relative to the noses (7) are flush with the recesses (8), thus allowing the rolling elements (4) to exit the V-shaped notches (9) of the inner sleeve (10) into the recesses (8) of the outer sleeve (1) while spreading the snap sleeve (3) when a maximally permissible torque is reached, thereby uncoupling the inner sleeve (10), and
- a torque applied to the outer sleeve (1) in the unscrewing direction presses the other side of the noses (7) against the other wall of the recesses (8) into a position in which the rolling elements (4) are flush with the webs remaining between the recesses (8), thus preventing the rolling elements (4) from exiting the V-shaped notches (9) of the inner sleeve (10), thereby coupling the inner sleeve (10), wherein
- the shaft (11) is provided at its tip with a cone (12) supported in a bearing cap.

2. The torque limiter as claimed in claim 1, wherein the rotational fixing between the outer sleeve (1) and the snap sleeve (3) is effected by the provision of a drive element (2) on the inner wall of the outer sleeve (1), said drive element protruding radially inwardly and engaging in the slot in the snap sleeve (3).

3. The torque limiter as claimed in claim 1 or 2, wherein the rolling elements (4) are pins and the rolling element receiving areas (5) are slots.

4. The torque limiter as claimed in one of the preceding claims, wherein the snap sleeve (3) is produced from a polyetheretherketone (PEEK).

## Revendications

1. Limiteur de couple, en particulier pour un tournevis chirurgical, avec
- un manchon extérieur (1), un manchon à encliquetage (3) disposé dans le manchon extérieur de manière à être bloqué en rotation ; une cage (6) à éléments roulants disposée à l'intérieur de celui-ci, un manchon intérieur (10) disposé à l'intérieur de celle-ci et un arbre (11) de transmission de force reçu par ce dernier de manière à être bloqué en rotation,
- la paroi intérieure du manchon extérieur (1) présentant des d'évidements (8) qui s'étendent parallèlement à l'axe,
- la cage (6) à éléments roulants présentant une pluralité de zones (5) de réception d'éléments roulants, dont chacune contient un élément roulant (4) et un nombre d'ergots (7) correspondant au nombre d'évidements (8), venant en engagement dans les évidements (8), la largeur des ergots (7) étant inférieure à la largeur libre des évidements (8), et
- le manchon intérieur (10) présentant une pluralité de rainures (9) en forme de V s'étendant parallèlement à l'axe, servant à recevoir les éléments roulants (4),
de sorte
- qu'un couple appliqué sur le manchon extérieur (1) dans le sens du vissage presse les côtés des ergots (7) contre l'une des parois des évidements (8) dans une position dans laquelle les éléments de roulement (4), qui sont disposés de manière légèrement décalée par rapport aux ergots (7), sont alignés avec les évidements (8) et, lorsqu'un couple maximal admissible est atteint, permettent ainsi la sortie des éléments de roulement (4) hors des rainures (9) en forme de V du manchon intérieur (10) dans les évidements (8) du manchon extérieur (1) tout en dilatant le manchon à encliquetage (3), de sorte que le manchon intérieur (10) soit découplé, et
- qu'un couple appliqué au manchon extérieur (1) dans le sens du dévissage presse les autres côtés des ergots (7) contre l'autre des parois des évidements (8) dans une position dans laquelle les éléments roulants (4) sont alignés avec les entretoises restant entre les évidements (8), faisant ainsi qu'un échappement des éléments roulants (4) des encoches en forme de V (9) du manchon intérieur (10) soit empêché, de sorte que le manchon intérieur (10) soit accouplé,
- l'arbre (11) étant muni à son extrémité d'un cône (12) monté dans un capuchon de palier.

2. Limiteur de couple selon la revendication 1, **caractérisé en ce que** l'empêchement de rotation entre le manchon extérieur (1) et le manchon à encliquetage (3) est réalisé par la mise en place d'un organe d'entraînement (2) prévu sur la paroi intérieure du manchon extérieur (1), faisant saillie radialement vers l'intérieur, qui vient en engagement dans la fente du manchon à encliquetage (3).

3. Limiteur de couple selon la revendication 1 ou la revendication 2, **caractérisé en ce que** les éléments de roulement (4) sont des aiguilles et les récepteurs d'éléments de roulement (5) sont des fentes.

4. Limiteur de couple selon l'une des revendications précédentes, **caractérisé en ce que** le manchon à encliquetage (3) est constitué d'un polyéther éther cétone (PEEK).
